# EUROPEAN PATENT APPLICATION

(11) **EP 3 892 360 A1**
(43) Date of publication of application: **13.10.2021**
(21) Application number: 20208228.5
(22) Date of filing: 17.11.2020
(51) Int. Cl.: B01D 53/88, A61L 9/20

(54) **AIR PURIFICATION MODULE AND AIR PURIFIER FORMED THEREOF**

(30) Priority: 06.12.2019 TW 108144739
(71) Applicant: IADIY TECHNOLOGY Ltd., Taipei City 106 (TW)
(72) Inventor: HU, DI-SHENG, 800 Kaohsiung City (TW)
(74) Representative: Lang, Christian

(57) **Abstract**

An air purification module and an air purifier formed thereof are revealed. An air purification module is a standardized module formed by an enclosing casing, at least one ultraviolet light emitting diode (UV LED) and at least one photocatalyst filter. While in use, the UV LED is electrically connected to the power source to emit UV light and drive the photocatalyst filter to be in an activated state for decomposition of bacteria, removal of odors and absorption of particulate pollutants in air flowing in, through and out of an axial air passage inside the enclosing casing. Thereby good air purification is achieved. The air purifier includes a housing with a number of air purification module mounted therein, corresponding fans, and at least one power unit. Thereby the use efficiency of the air purifier is increased and consumers have more choices.

## Description

### BACKGROUND OF THE INVENTION

The present invention relates to an air purification module and an air purifier formed thereof, especially to a standardized air purification module which is composed of an enclosing casing, at least one ultraviolet light emitting diode, and at least one photocatalyst filter, an air purifier which is constructed by a housing with proper design, a certain number of air purification modules mounted in the housing, at least one fan and at least one power unit.

Air pollution has a significant influence on human health so that air purifiers have become more popular and the need for air purifiers is increasing day by day. Now there are a lot of air-purification related devices available. For example, a device revealed in Chinese Utility Patent App. No. 201721649857.1 provides functions of pathogen-killing light and air purification by ultraviolet and by photocatalyst respectively. However, most of prior arts in the field are an air purification product manufactured at the manufacturer end able to be selected at the consumer end. The internal structures of the respective air purification products, including the ultraviolet structure and the photocatalyst structure, are installed separately. That means the respective manufacturers have their own design and specifications so that the products don't have standardized specifications and modular components. Thus, the production cost of the manufacturer is unable to be reduced effectively. The use efficiency (air purification efficiency) of the air purification product is also deteriorated or limited. Moreover, the air purification product may be unable to satisfy users' requirements. The air purification device should be selected according to the space/the place the product is in, or the way the product is used (fixed or portable in use). For example, the air purification device used on a small table/in a small room is unable to be applied to a conference table for many people/in a large space. The design of the air purification device suitable for the vehicle is different from that of the portable air purification device used in baby strollers. These lead to trouble in purchases or applications at the consumer end. Therefore, there is room for improvement and there is a need to provide an air purification module and an air purifier formed thereof with novel design in order to solve the above problems effectively.

### SUMMARY OF THE INVENTION

Therefore it is a primary object of the present invention to provide an air purification module which is mass produced in a standardized and modularized way, and an air purifier which is constructed by a certain number of the air purification modules while both the number and the structure of the air purification modules are selectable. Thereby both manufacturing efficiency and efficiency in use of the air purification module and the air purifier are improved.

In order to achieve the above object, an air purification module according to the present invention includes an enclosing casing, at least one ultraviolet light emitting diode (UV LED) and at least one photocatalyst filter. The enclosing casing has an enclosing wall with a certain axial width extending along a central axis thereof. A first section and a second section are formed on two ends of the axial width respectively and parallel to each other. The axial air passage is formed inside the enclosing wall between the first section and the second section for allowing external air to flow into the axial air passage from one of the sections, pass through the axial air passage, and then flow out through the other section. The respective UV LEDs are arranged at the inner surface of the enclosing wall and emitting UV light toward the axial air passage after being electrically connected to a power source. The respective photocatalyst filters are mounted on at least one of the first and the second sections (either the first section or the second section) and activated by UV light emitted from the respective UV LEDs. While in use, the respective UV LEDs are electrically connected to the power source to emit UV light and activate the respective photocatalyst filters for decomposition of bacteria, removal of odors and absorption of particulate pollutants in the air flowing in, through and out of the axial air passage. Thereby multiple functions of the air purification module are achieved.

Preferably, the UV LED can emit ultraviolet light C (UVC) with the wavelength ranging from 100 nanometer (nm) to 280 nm.

Preferably, a section of the enclosing casing can be a rectangular hollow section, a round hollow section, a triangular hollow section, a pentagonal hollow section, or a hexagonal hollow section (all not shown in figures)

Preferably, a fan is disposed on either the first section or the second section of the enclosing casing. Under the action of the fan, the flow velocity per unit time or flow rate per unit time of the air passed through the axial air passage can be accelerated. The fan can be an axial fan or a centrifugal fan.

Preferably, a multiple module assembly is formed by a plurality of the air purification modules arranged in different ways. For example, a plurality of the air purification modules are aligned with and stacked on one another to form an axially stacked multiple module assembly. Or each of the air purification modules is connected to one side of another air purification module and the air purification modules are arranged in parallel to form a parallel arranged multiple module assembly. Or the air purification modules are not only stacked axially but also arranged in parallel to one another to form an axially-stacked parallel-arranged multiple module assembly.

Preferably, at least one fan is mounted on one section of the outermost air purification module of the multiple module assembly. Thereby the flow velocity per unit time or flow rate per unit time of the air passed through the axial air passage of the respective air purification modules of the multiple module assembly can be accelerated under the action of the fan. The fan selection is based on the arrangements of or requirements for the multiple module assembly. The fan can be an axial fan or a centrifugal fan.

Preferably, at least one fastener is integrally extending from the enclosing casing and composed of a fastening portion and a fastened portion corresponding to each other. Thus the fastening portion of the fastener of one of the air purification modules and the fastened portion of another air purification module 1 are fastened and assembled with each other to form one part. One of the air purification modules can also be integrated and assembled with a fan by the fastening portion of the fastener thereof.

In order to achieve the above object, an air purifier according to the present invention includes a housing, at least one air purification module, at least one fan, and at least one power unit. The housing is provided with at least one cavity therein, at least one air-inlet portion disposed thereon and at least one air-outlet portion arranged thereon. Thereby external air flows into the respective cavities through the respective air-inlet portions and then flows out through the respective air-outlet portions. The respective air purification modules are mounted and fixed in the respective cavities while the respective fans are arranged at one section of the at least one air purification module so that the flow velocity per unit time or flow rate per unit time of the air passed through the axial air passage of the respective air purification modules can be accelerated under the action of the fans. The respective power units are set on the housing and electrically connected to the respective air purification modules and the respective fans for providing power to them. The power unit is provided with a switch used for turning on or off the respective air purification modules and the respective fans.

While the air purifier is in use, the respective power units supply power so that external air is drawn into the respective cavities through the respective air-inlet portions of the housing and flowing through the respective axial air passages of the respective air purification modules under the action of the respective fans for air purification. Then the air already been purified is exhausted through the respective air-outlet portions of the housing.

Preferably, the air purifier is applied to a desktop air purifier, a car air purifier, or a portable air purifier.

Preferably, the number of the air purification modules in the respective cavities is able to be selected according to requirements for the air purifier or one kind of the multiple module assembly can be used in the air purifier.

### BRIEF DESCRIPTION OF THE DRAWINGS

Fig. 1 is a schematic drawing showing a perspective view of an embodiment according to the present invention;
Fig. 2 is a schematic drawing showing a perspective view of another embodiment according to the present invention;
Fig. 3 is a schematic drawing showing a right side planar view of a further embodiment (including a fastening member) according to the present invention;
Fig. 4 is a schematic drawing showing a right side planar view of the embodiment in Fig. 3 assembled with an axial fan according to the present invention;
Fig. 5 is a schematic drawing showing a right side planar view of the embodiment in Fig. 4 assembled with an air purification module according to the present invention;
Fig. 6 is a schematic drawing showing a right side planar view of the embodiment in Fig. 5 assembled with one more air purification module according to the present invention;
Fig. 7 is a schematic drawing showing a right side planar view of an embodiment (in which a fan added is a centrifugal fan) according to the present invention;
Fig. 8 is a schematic drawing showing a right side planar view of the embodiment in Fig. 7 assembled with an air purification module according to the present invention;
Fig. 9 is a schematic drawing showing a right side planar view of the embodiment in Fig. 8 assembled with one more air purification module (a right side of an enclosing wall is hidden for showing internal structure of the conveniently) according to the present invention;
Fig. 10 is a schematic drawing showing a front planar view of a multiple module assembly formed by a plurality of air purification modules according to the present invention;
Fig. 11 is a schematic drawing showing a front planar view of another multiple module assembly formed by a plurality of air purification modules according to the present invention;
Fig. 12 is a perspective view of an embodiment according to the present invention;
Fig. 13 is a perspective view of the embodiment in Fig. 12 viewed from another angle according to the present invention;
Fig. 14 is a perspective view of another embodiment (axially stacked multiple module assembly) according to the present invention;
Fig. 15 is a schematic drawing showing a perspective view of another embodiment (axially stacked multiple module assembly with a plurality of fans) according to the present invention;
Fig. 16 is a schematic drawing showing a front planar view of a further embodiment (axially stacked and parallel arranged multiple module assembly) according to the present invention;
Fig. 17 is a schematic drawing showing a perspective view of a further embodiment (in which a fan added is a centrifugal fan) according to the present invention;
Fig. 18 is a schematic drawing showing a perspective view of a further embodiment (parallel arranged multiple module assembly) according to the present invention;
Fig. 19 is a schematic drawing showing an embodiment being applied to a desktop air purifier used indoors according to the present invention;
Fig. 20 is a schematic drawing showing an embodiment being applied to a car air purifier used in vehicles according to the present invention;
Fig. 21 is a schematic drawing showing a perspective view of an embodiment being applied to a portable air purifier according to the present invention;
Fig. 22 is a schematic drawing showing an embodiment being applied to a portable air purifier used in a baby stroller according to the present invention.

### DETAILED DESCRIPTION OF THE PREFFERED EMBODIMENTS

The structure and technical features of the present invention are described in detail with reference to the figures. The respective figures are only used to show structure and related functions of the present invention. Thus the size of the respective components in the figures are not drawn in actual scales and not intended to limit the present invention.

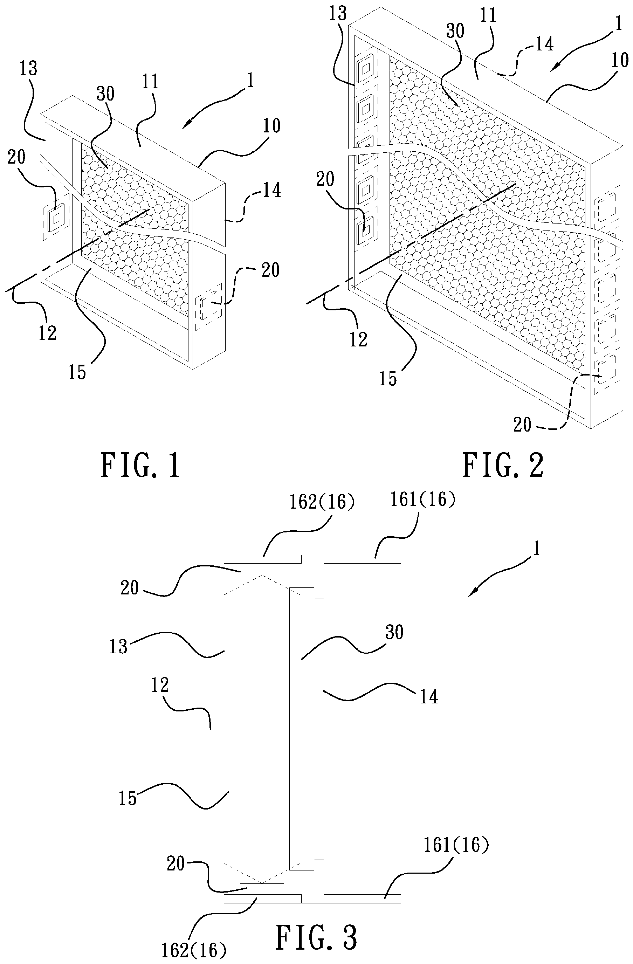

Refer to Fig. 1-3, an air purification module 1 of the present invention includes an enclosing casing 10, at least one ultraviolet light emitting diode (UV LED) 20 and at least one photocatalyst filter 30.

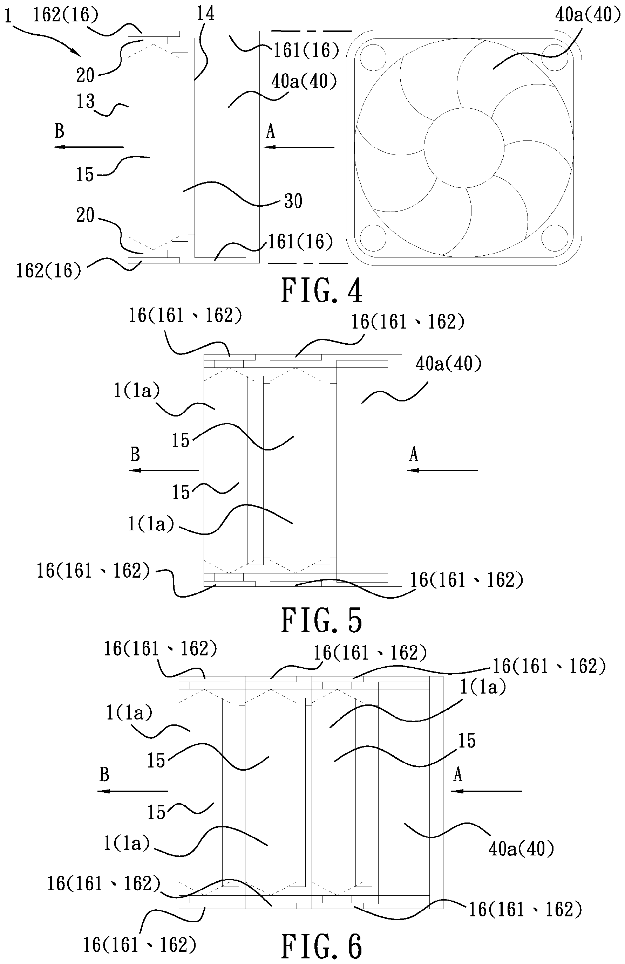

The enclosing casing 10 consists of an enclosing wall 11 with a certain axial width extending along a central axis 12 thereof, a first section 13, a second section 14, and an axial air passage 15. The first section 13 and the second section 14 are formed on two ends of the axial width respectively and parallel to each other. The axial air passage 15 is formed inside the enclosing wall 11 between the first section 13 and the second section 14 and used for allowing external air to flow into the axial air passage 15 from one of the sections 13, 14 (either the first section 13 or the second section 14) (as the arrow A in Fig. 4-9 indicates), pass through the axial air passage 15, and then flow out through the other section 13, 14 (as the arrow B in Fig. 4-9 indicates).

As shown in Fig. 1 and Fig. 2, the enclosing casing 10 is a rectangular casing which looks like a rectangular hollow tube with a rectangular hollow section. The section of the enclosing casing 10 can also be in other shapes such as circular/round hollow section, a triangular hollow section, a pentagonal hollow section, or a hexagonal hollow section (all not shown in figures).

The respective UV LEDs 20 are arranged at the inner surface of the enclosing wall 11 and emitting UV light toward the axial air passage 15 after being electrically connected to a power source. In a preferred embodiment, the UV LED 20 can emit ultraviolet light C (UVC, also called shortwave ultraviolet, ultraviolet germicidal irradiation) with the wavelength ranging from 100 nanometer (nm) to 280 nm preferably. It can also emit UV light with the wavelength ranging from 100 nm to 400 nm optionally. For example, the UV light emitted can be ultraviolet B (UVB) rays having the wavelength in the range of 315-280 nm for saving the cost. Yet the UVC is more capable of killing pathogens compared with UVB.

The respective photocatalyst filters 30 are disposed on at least one of the sections 13, 14 (either the first section 13 or the second section 14). As shown in Fig. 1-3, the photocatalyst filter 30 is arranged at the second section 14 in this embodiment. When being energized by UV light emitted from the respective UV LEDs 20, the respective photocatalyst filters 30 are activated to work. While the air purification module 1 is in use, the respective UV LEDs 20 are electrically connected to the power source in order to emit UV light. Then the respective photocatalyst filters 30 are driven and activated by the UV light for decomposition of bacteria, removal of odors and absorption of particulate pollutants in the air flowing in, through and out of the axial air passage 15. Thereby multiple functions of the air purification module 1 are achieved. The photocatalyst filter 30 can be a nano silver and photocatalyst filter. The photocatalyst filter 30 can also be coated with titanium dioxide.

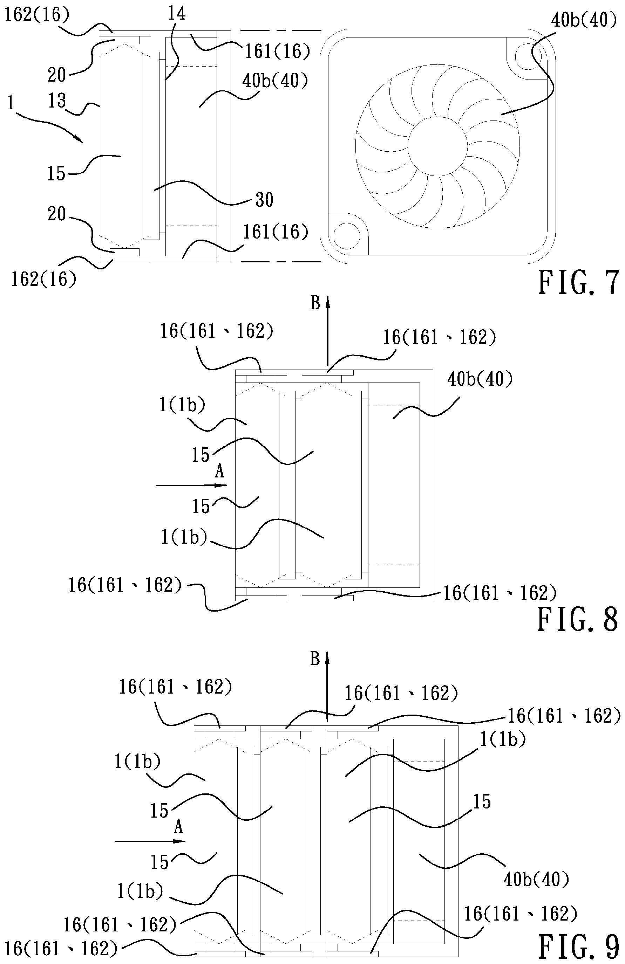

Moreover, a fan 40 is disposed on the first section 13 or the second section 14 of the enclosing casing 10. Refer to Fig. 4-7, the fan 40 is arranged at the second section 14 of the enclosing casing 10 in these embodiments. Under the action of the fan 40, the flow velocity per unit time or flow rate per unit time of the air passed through the axial air passage 15 can be accelerated. The fan 40 includes an axial fan 40a (as shown in Fig. 4) and a centrifugal fan 40 b (as shown in Fig. 7).

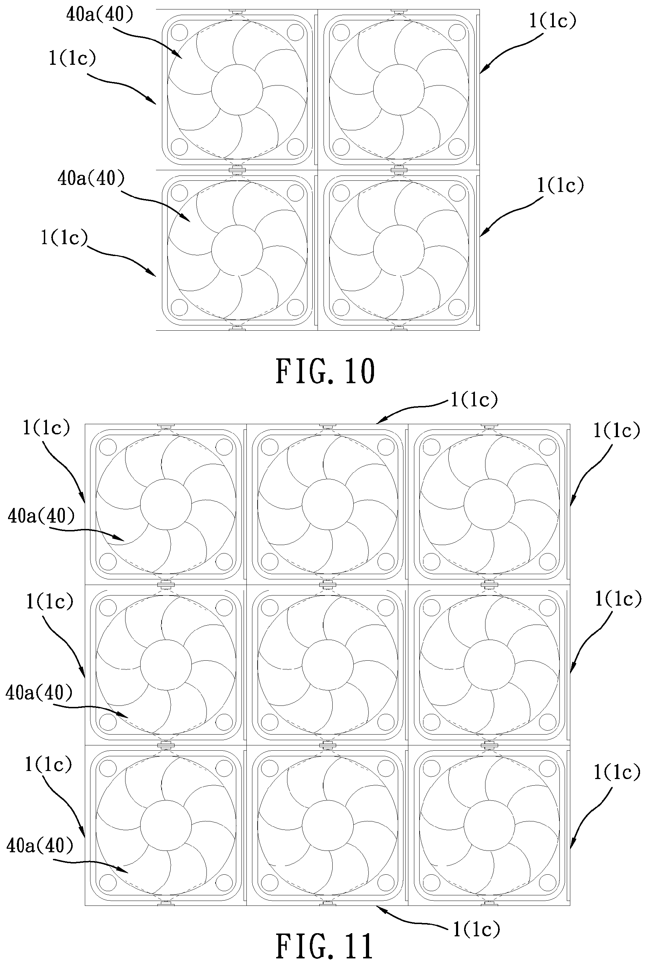

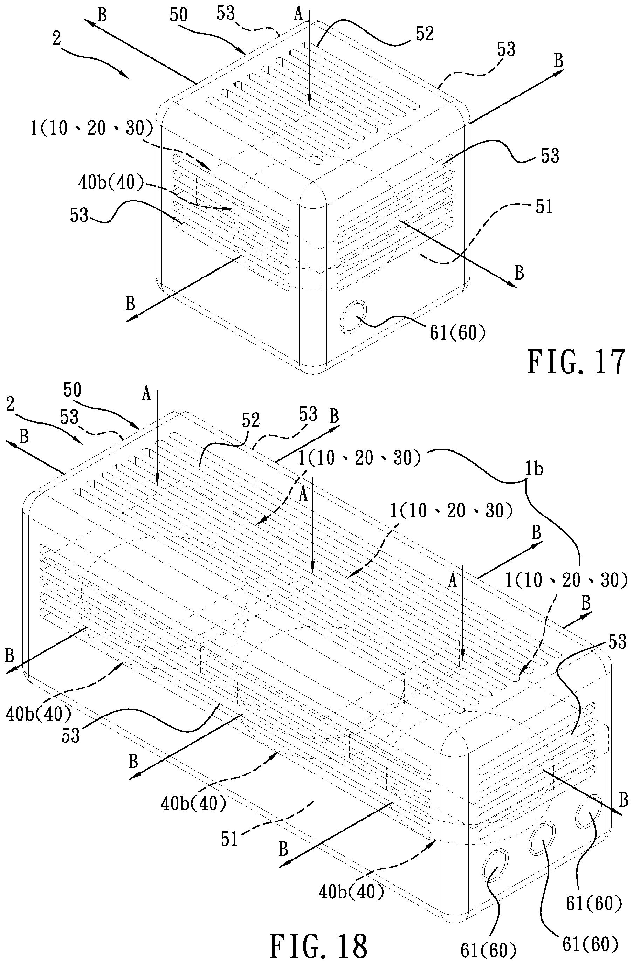

Refer to Fig. 5-6, Fig. 8-9, Fig. 10-11 and Fig. 18, a plurality of air purification modules are assembled in different ways to form a multiple module assembly at the manufacturer end. The multiple module assembly has the following different combinations. As shown in Fig. 5, Fig. 6, Fig. 8, and Fig. 9, two or three air purification modules 1 are stacked axially. That is to say, a plurality of air purification modules 1 are aligned with and stacked on one another to form an axially stacked multiple module assembly 1a. Also refer to Fig. 10 (four air purification modules 1), Fig. 11 (nine air purification modules 1), and Fig. 18 (three air purification modules 1), a plurality of air purification modules 1 are arranged in parallel. Each of the air purification modules 1 is connected to one side of another air purification module 1 in turn and the air purification modules 1 are disposed in parallel to form a parallel arranged multiple module assembly 1b, as shown in Fig. 18 (three purification modules 1). Refer to the embodiment in Fig. 10, it has a multiple layers each of which includes four air purification modules 1 while the embodiment in Fig. 11 has a multiple layers each of which is provided with nine air purification modules 1. As to the embodiments in Fig. 10 and Fig. 11, a plurality of air purification modules 1 are not only stacked axially but also arranged in parallel to one another to form an axially-stacked parallel-arranged multiple module assemblies 1c. Moreover, the multiple module assemblies 1a shown in Fig. 5, Fig. 6, Fig. 8 and Fig. 9 provide improvement of air purification intensity, thus able to be applied to places with poor air quality. The multiple module assembly 1b shown in Fig. 18 also brings higher purification intensity, able to be applied to places full of people for quicker improvement of air quality. As to the multiple module assemblies 1c shown in Fig. 10 and Fig. 11, it is good for improving both intensity and speed of air purification. The multiple module assembly 1c is able to be applied to places which are full of people and having poor air quality for improvement of air quality.

Refer to Fig. 3-9, at least one fastener 16 which includes a fastening portion 161 and a corresponding fastened portion 162 is integrally extending from the enclosing casing 10. Thus, the fastening portion 161 of the fastener 16 of one of the air purification modules 1 and the fastened portion 162 of another air purification module 1 are fastened and assembled with each other to form one part. Moreover, one of the air purification modules 1 can also be integrated and assembled with a fan 40 by the fastening portion 161 of the fastener 16 thereof.

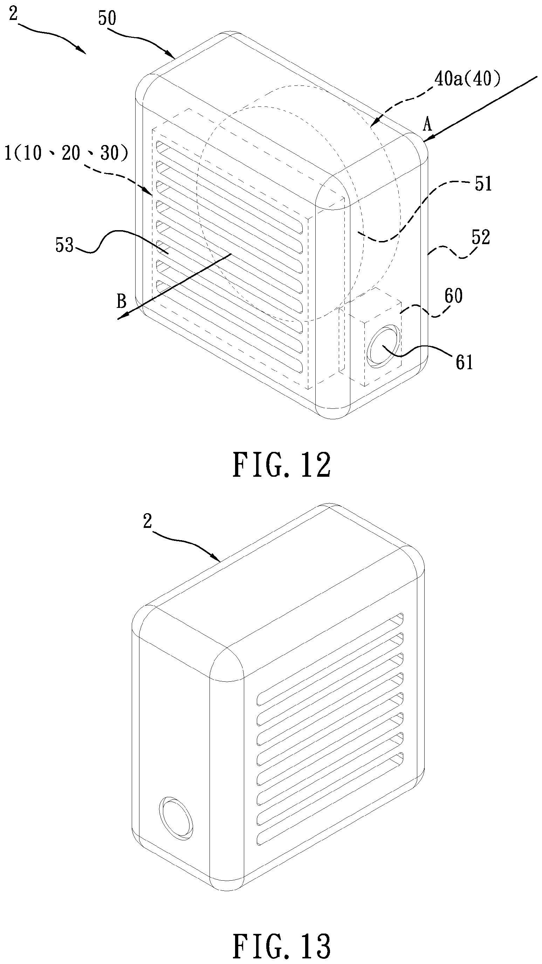

Refer to Fig. 12-18, an air purifier 2 formed by the air purification modules 1 is revealed. The air purifier 2 is composed of a housing 50, at least one air purification module 1, at least one fan 40, and at least one power unit 60. The housing 50 is a body which includes at least one cavity 51 therein, at least one air-inlet portion 52 disposed thereon and at least one air-outlet portion 53 arranged thereon. Thereby external air flows into the respective cavities 51 through the respective air-inlet portions 52 (as the arrow A in Fig. 12, Fig. 14 and Fig. 22 indicates) and then flows out through the respective air-outlet portions 53 (as the arrow B in Fig. 12, Fig. 14 and Fig. 22 indicates).

The respective air purification modules 1 are mounted and fixed in the respective cavities 51 while the respective fans 40 are arranged at one section of the respective air purification modules 1 (such as the first section 13 or the second section 14) so that the flow velocity per unit time or flow rate per unit time of the air passed through the axial air passage 15 can be accelerated under the action of the fan 40. The respective power units 60 are set on the housing 50 and electrically connected to the respective air purification modules 1 and the respective fans 40 for providing power to them. The power unit 60 is provided with a switch 61 for turning on or off the respective air purification modules 1 and the respective fans 40.

While the air purifier 2 is in use, the respective power units 60 provide power so that the air purifier 2 draws external air into the respective cavities 51 through the respective air-inlet portions 52 of the housing 50 and purify the external air flowing through the respective axial air passages 15 of the respective air purification modules 1 under the action of the respective fans 40. Then the purified air is exhausted through the respective air-outlet portions 53 of the housing 50.

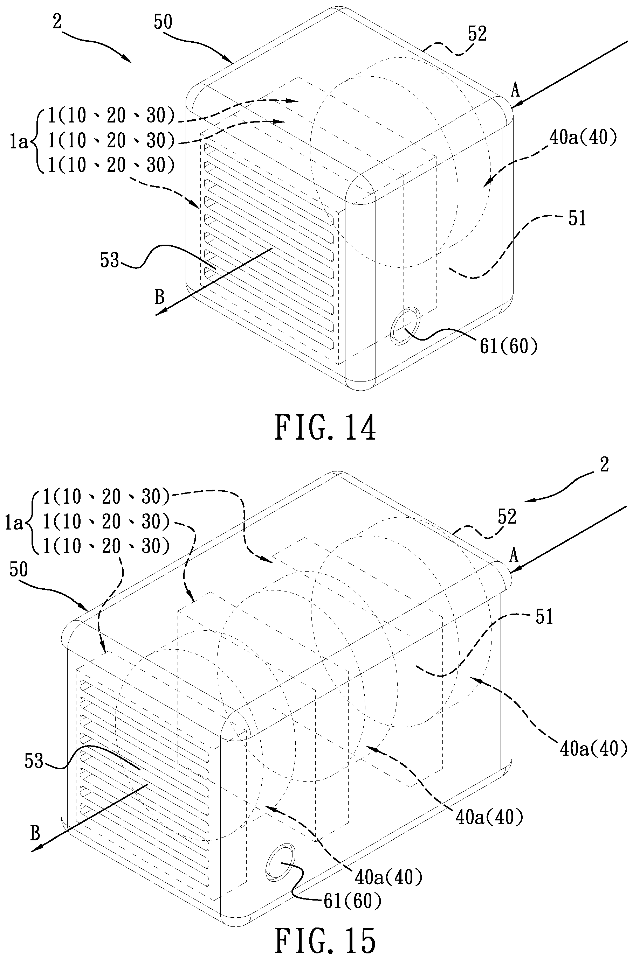

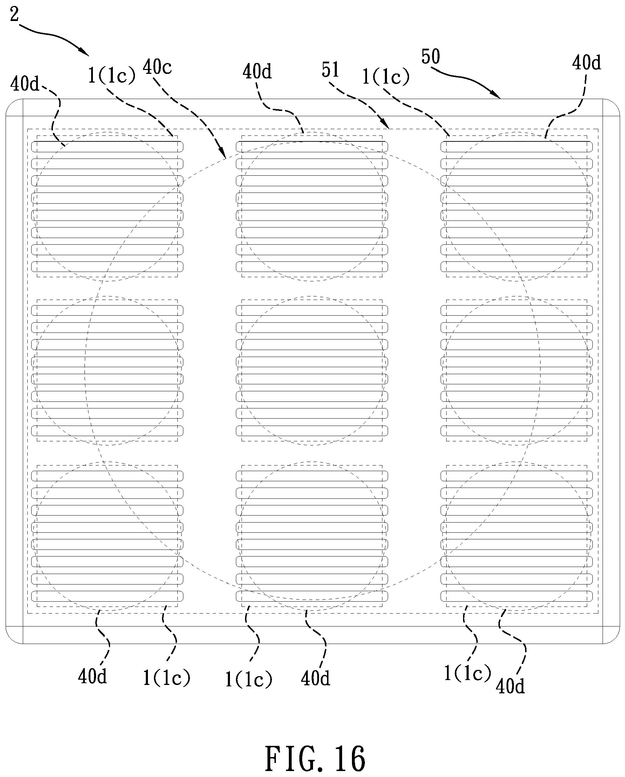

Moreover, the respective fans 40 can be arranged in different ways because the air purification modules 1 mounted in the cavity 51 of the air purifier 2 are designed to have various multiple module assemblies 1a, 1b, 1c. For example, a fan 40 (preferably an axial fan 40a) is disposed on one section (such as the second section 14) of the outermost air purification module 1 among the two or three axially stacked air purification modules 1 in the embodiments shown in Fig. 5-6 and Fig. 8-9. Refer to the embodiment shown in Fig. 15, a fan 40 (preferably an axial fan 40a) is disposed on the second section 14 of each of the three air purification modules 1 axially stacked. In this embodiment, there are three air purification modules land three fans 40. Refer to Fig. 16, a large-scale fan 40c (able to be an axial fan 40a or a centrifugal fan 40b) is arranged at the second sections 14 of each of the nine air purification modules 1 arranged parallel to one another. Or a small fan 40d (preferably an axial fan 40a) smaller than the air purification module 1 is disposed on each of the second sections 14 of the respective air purification modules 1. Thus there are two types of the arrangement of the fans in this embodiment. The first is that only a single large fan 40c is used while the other type uses nine small fans 40d. As to the embodiment shown in Fig. 18, a fan 40 (the centrifugal fan 40b in the figure but not limited) is disposed on each of the second sections 14 of the three air purification modules 1 arranged in parallel. Thus there are three air purification modules 1 and three fans 40 (such as the centrifugal fans 40b) in this embodiment. While in use, the flow velocity per unit time or flow rate per unit time of the air passed through the axial air passage 15 of the respective air purification modules 1 which form the multiple module assembly (1a, 1b, 1c) can be accelerated under the action of the fans 40 (40c, 40d). The fan 40 can be selected from the axial fan 40a and the centrifugal fan 40b, depending on the assembly pattern or the cost of the respective air purification modules 1 of the multiple module assembly (1a, 1b, 1c).

Furthermore, take the embodiment shown in Fig. 18 as an example. In this embodiment, there are three air purification modules 1 arranged in parallel and each of the air purification modules 1 is provided with a fan 40 (the centrifugal fan 40b is used). Three sets of power units 60 and switches 61 are provided for turning on/off the three parallel arranged air purification modules 1 and the fans 40 correspondingly. While in use, users (consumer end) can choose to turn on one, two or all of the three parallel arranged air purification modules 1 (and the corresponding fans 40) according to their needs (such as the size of the place or the number of the personnel required). Thereby the air purifier 2 shown in Fig. 18 can have higher efficiency in use.

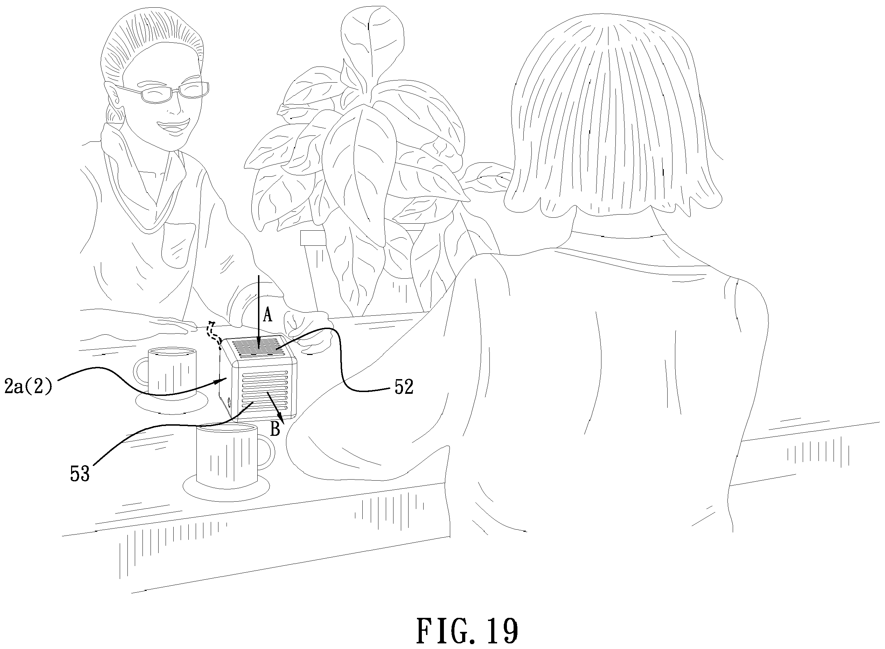

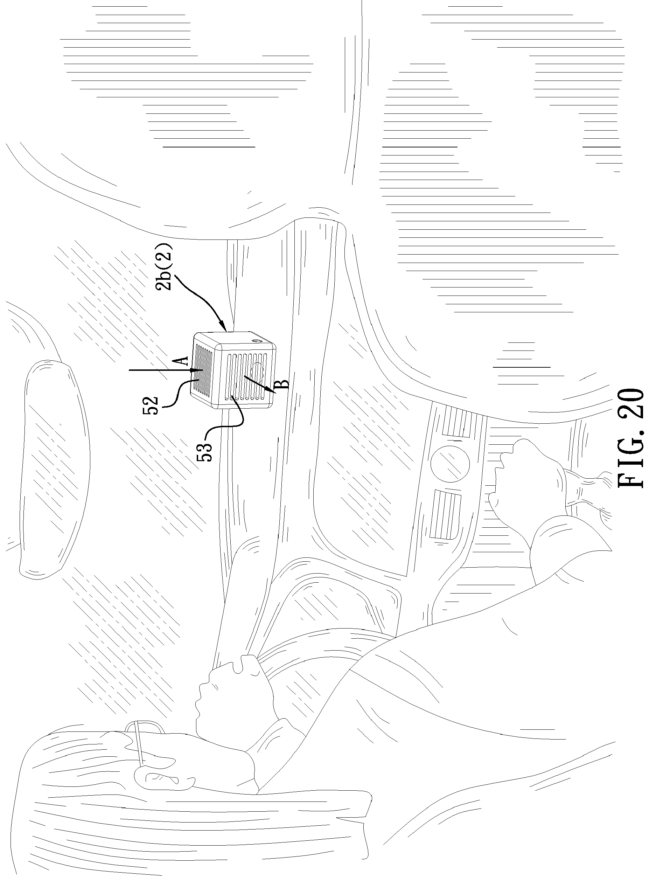

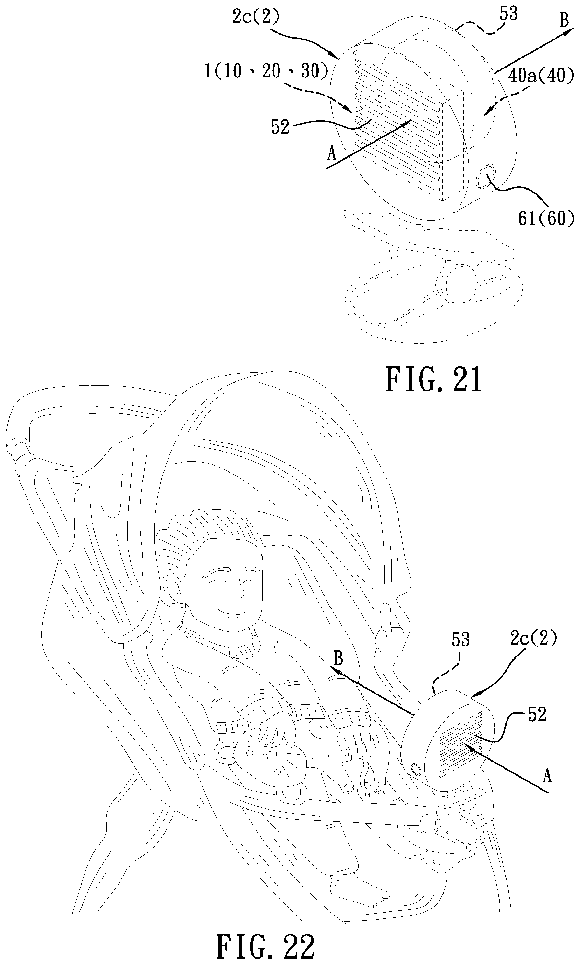

Refer to Fig. 19, Fig. 20 and Fig. 22, the air purifier 2 can have the following types according to different places they are used. As shown in Fig. 19 and Fig. 20, a desktop air purifier 2a and a car air purifier 2b are revealed respectively. Refer to Fig. 21 and Fig. 22, a portable air purifier 2c is revealed. The desktop air purifier 2a shown in Fig. 19 can be used in private or public space such as a small room, a private office or a shared office. As shown in Fig. 19, the desktop air purifier 2a is set on a surface of a table between two people to improve air quality at the place effectively. Even one of the people has a cold or allergies, the desktop air purifier 2a makes the environment more safe and healthy. As to the car air purifier 2b shown in Fig. 20, it is fixed on the cars by a fixing member such as a suction cup. Refer to Fig. 20, the car air purifier 2b is fixed on a car dashboard to improve air quality in the car effectively. The portable air purifier 2c shown in Fig. 21 and Fig. 22 can also be fixed on a baby stroller by a fastener. Refer to Fig. 21 and Fig. 22, the portable air purifier 2c is fixed on a front rail of the baby stroller for improving air in front of the baby or around the baby stroller so that the air the baby is breathing is cleaner and safer.

The air purification modules 1 of the present invention are mass produced in a in a standardized and modularized way at the manufacturer's site. Then a housing 50 of an air purifier 2 is designed according to the air purification modules 1 produced by the same or different manufacturer. Next a certain number of the air purification modules 1 which form the multiple module assemblies 1a, 1b and 1c are used in combination with the housing 50 to construct an air purifier 2. For example, the manufacturers can determine the number of the air purification modules 1 or select one of the multiple module assemblies 1a, 1b and 1c going to be mounted in the respective cavities 51 according to user's requirements. Thereby the efficiency in manufacturing and use of the air purification modules 1 or the air purifier 2 is significantly increased.

Compared with conventional techniques, the air purification module and the air purifier formed thereof according to the present invention have the following advantages:
(1) The present air purification module 1 provides both ultraviolet sterilization and photocatalyst purification so that the use efficiency of the air purification module 1 is improved.
(2) The air purification modules 1 of the present invention are mass produced in a in a standardized and modularized way so that the production cost of the air purification module 1 or the air purifier 2 is effectively reduced. This is beneficial to both the manufacturer end and the consumer end.
(3) The present air purifier 2 is designed and constituted by the standardized and modularized air purification modules 1 so that the air purifier 2 is easier to be mass produced. The use efficiency (air purification efficiency) of the respective air purifiers 2 can be selected freely or operated (such as the embodiment in Fig. 18) by users (consumer end) according to their needs (such as the size of the place or the number of the personnel required). Thus the cost of the air purifier 2 can be effectively decreased and this is beneficial to both the manufacturer end and the consumer end.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalent.

## Claims

1. An air purification module comprising:
an enclosing casing which includes an enclosing wall with an axial width extending along a central axis thereof, a first section and a second section are formed on two ends of the axial width respectively and parallel to each other, and an axial air passage formed inside the enclosing wall between the first section and the second section for allowing external air to flow into the axial air passage from one of the first and the second sections, pass through the axial air passage, and then flow out through the other section;
at least one ultraviolet light emitting diode (UV LED) which is arranged at an inner surface of the enclosing wall and emitting ultraviolet (UV) light toward the axial air passage after being electrically connected to a power source; and
at least one photocatalyst filter disposed on at least one of the first and the second sections; wherein the photocatalyst filter is activated to work by the UV light emitted from the UV LED;
wherein the UV LED is electrically connected to the power source to emit UV light and drive the photocatalyst filter to be in an activated state for decomposition of bacteria, removal of odors and absorption of particulate pollutants in the air flowing in, through and out of the axial air passage.

2. The air purification module as claimed in claim 1, wherein UV LED emits ultraviolet light C (UVC) with the wavelength ranging from 100 nanometer (nm) to 280 nm.

3. The air purification module as claimed in claim 1, wherein a section of the enclosing casing is selected from the group consisting of a rectangular hollow section, a round hollow section, a triangular hollow section, a pentagonal hollow section, and a hexagonal hollow section.

4. The air purification module as claimed in claim 1, wherein a fan is disposed on one of the first and the second sections of the enclosing casing; flow velocity per unit time or flow rate per unit time of the air passed through the axial air passage is accelerated under action of the fan; wherein the fan is either an axial fan or a centrifugal fan.

5. The air purification module as claimed in claim 1, wherein a multiple module assembly is formed by a plurality of the air purification modules arranged in different ways and is selected from the group consisting of an axially stacked multiple module assembly formed by a plurality of the air purification modules aligned with and stacked on one another, a parallel arranged multiple module assembly formed by a plurality of the air purification modules each of which is connected to one side of the adjacent air purification modules and arranged in parallel to one another, and an axially-stacked parallel-arranged multiple module assembly formed by a plurality of the air purification modules which are not only stacked axially but also arranged in parallel to one another.

6. The air purification module as claimed in claim 5, wherein at least one fan is disposed on one of the first and the second sections of the outermost air purification module of the multiple module assembly; flow velocity per unit time or flow rate per unit time of the air passed through the axial air passage of the respective air purification modules of the multiple module assembly is able to be accelerated under action of the fan; wherein the fan is either axial fan or a centrifugal fan.

7. The air purification module as claimed in claim 6, wherein at least one fastener is integrally extending from the enclosing casing and composed of a fastening portion and a fastened portion corresponding to each other; the fastening portion of the fastener of one of the air purification modules and the fastened portion of another air purification module are fastened and assembled with each other to form one part; one of the air purification modules is able to be fastened and assembled with the fan by the fastening portion of the fastener thereof.

8. An air purifier formed by at least one air purification module comprising:
a housing which includes at least one cavity therein, at least one air-inlet portion disposed thereon and at least one air-outlet portion arranged thereon for allowing external air to flow into the cavity through the air-inlet portion and then flow out through the air-outlet portion;
at least one air purification module mounted and fixed in the cavity;
at least one fan arranged at one section of the air purification module so that flow velocity per unit time or flow rate per unit time of air passed through an axial air passage of the air purification module is able to be accelerated under action of the fan; and
at least one power unit arranged at the housing and electrically connected to the air purification module and the fan for providing power to the air purification module and the fan; wherein the power unit is provided with a switch used for turning on or off the air purification module and the fan;
wherein the power unit supplies power so that external air is drawn into the cavity through the air-inlet portion of the housing and flowing through the axial air passage of the air purification module under action of the fan for air purification; then air already purified is exhausted through the air-outlet portion of the housing.

9. The air purifier as claimed in claim 8, wherein the air purifier is further applied to a desktop air purifier, a car air purifier, and a portable air purifier.

10. The air purifier as claimed in claim 8, wherein the number of the air purification module in the cavity of the air purifier is able to be selected according to requirements for the air purifier while in use.
